# FASCICULE DE BREVET EUROPEEN

(11) **EP 1 838 243 B1**
(45) Date de publication et mention de la délivrance du brevet: **27.04.2016**
(21) Numéro de dépôt: 05798451.0
(22) Date de dépôt: 18.08.2005
(51) Int. Cl.: A61F 2/16

(54) **DISPOSITIF DE CHARGEMENT D'UNE LENTILLE INTRAOCULAIRE DANS UNE CARTOUCHE D'INJECTION**
VORRICHTUNG ZUM LADEN EINER INTRAOKULARLINSE IN EINE INJEKTIONSKARTUSCHE
DEVICE FOR LOADING AN INTRAOCULAR LENS INTO AN INJECTION CARTRIDGE

(30) Priorité: 13.09.2004 FR 0409698
(43) Date de publication de la demande: 03.10.2007
(73) Titulaire: SIFI MEDTECH S.r.l., Aci Sant'Antonio, Frazione Lavinaio (IT)
(72) Inventeur: MEUNIER, Patrick, 00046 Grottaferrata (FR); LE PAIR, Jean-Marc, 56400 Brech (FR)
(74) Mandataire: Gerli, Paolo
(86) Numéro de dépôt international: PCT/FR2005/002096
(87) Numéro de publication internationale: WO 2006/030082

(56) Documents cités:
- EP-A- 1 338 254
- WO-A-99/62436
- WO-A-03/045285
- US-A- 5 944 725
- US-A1- 2003 195 522

## Description

La présente invention concerne un dispositif de chargement d'une lentille intraoculaire souple dans une cartouche d'injection. La présente invention concerne plus particulièrement un dispositif de chargement servant de support de conditionnement d'une lentille intraoculaire souple avant utilisation, ainsi qu'une cartouche d'injection spécifique utilisable avec ledit dispositif de chargement et un ensemble formé dudit dispositif de chargement et d'une cartouche d'injection.

Les lentilles intraoculaires (LIO) sont utilisées pour le remplacement du cristallin naturel de l'oeil affecté par la cataracte. L'extraction du cristallin naturel et son remplacement par une lentille intraoculaire se fait de façon chirurgicale. Avec l'apparition de la méthode chirurgicale utilisant la phacoémulsification du cristallin naturel, il a été proposé des lentilles intraoculaires réalisées en matériaux souples et pliables afin qu'elles puissent être introduites sous forme pliée dans l_{'}oeil par une petite incision. Cette introduction de la lentille intraoculaire dans l'oeil est généralement réalisée au moyen d'un système d'injection constitué d'un injecteur manuel de type seringue, réutilisable ou à usage unique, et d'une cartouche d'injection à usage unique. De manière classique, la cartouche d'injection comprend une chambre de réception de la lentille se prolongeant par une canule d'injection, la chambre de réception étant formée de deux demi-tubes reliés par une charnière, chaque tube étant muni d'une ailette pour permettre la fermeture de la chambre de réception. Après la mise en place d'une lentille conditionnée dans un emballage stérile dans la chambre en position ouverte à l'aide d'un instrument tel qu'une pince, puis pliage de la lentille par fermeture de la chambre, la cartouche est placée dans un injecteur approprié, comprenant un corps cylindrique dans lequel un piston est monté mobile. L'injection de la lentille intraoculaire pliée dans la cartouche est alors effectuée de manière mécanique ou hydraulique en actionnant le piston, après insertion de l'extrémité distale de la canule dans l'oeil.

Pour faciliter le transfert de la lentille intraoculaire à la cartouche d'injection des lentilles, il a été proposé dans le document EP 1 173 115, un dispositif de chargement d'une lentille dans la chambre de réception en position ouverte d'une cartouche d'injection, le dispositif étant formé d'un premier système support apte à recevoir une cartouche d'injection et à maintenir sa chambre de réception dans une position ouverte et un second système support apte à tenir une lentille intraoculaire. Les deux systèmes coopèrent pour permettre le transfert de la cartouche dans une position ouverte, à plat, sur le second système support, le second système support étant ensuite plié pour transférer la lentille intraoculaire dans la chambre de réception et permettre le retrait manuel de la cartouche du second support, puis la chambre de réception est fermée manuellement. Les deux systèmes supports sont destinés à un usage unique et servent également de systèmes d'emballage de la cartouche et de la lentille avant utilisation.

Le document EP-A- 1 338 254 décrit un dispositif de chargement d'une lentille intraoculaire souple mais ne décrit pas de cartouche; la lentille est directement chargée dans l'instrument d'insertion, qui store ladite lentille durant le transport.

Le but de la présente invention est de proposer un nouveau dispositif de chargement d'une lentille intraoculaire souple dans une cartouche d'injection comprenant une chambre de réception d'une lentille et une canule d'injection, qui soit simple de conception et simple à utiliser.

Dans ce but, la présente invention a pour objet un dispositif de chargement d'une lentille caractérisé en ce qu'il comprend un élément tubulâire ayant un passage interne, ledit passage comprenant un tronçon de réception d'une lentille à l'état non plié, un tronçon intermédiaire tronconique de pliage et un tronçon d'extrémité apte à recevoir amovible une cartouche d'injection d'une manière telle que le tronçon intermédiaire tronconique de pliage débouche sur la chambre de réception de la cartouche d'injection, un élément support monté dans ledit tronçon de réception, apte à supporter une lentille dans un état non plié, et un poussoir comprenant une tige montée mobile dans ledit passage interne, apte à pousser une lentille disposée dans le tronçon de réception à travers le tronçon intermédiaire tronconique de pliage pour plier ladite lentille progressivement, puis dans la chambre de réception d'une cartouche d'injection placée dans le tronçon d'extrémité.

Le dispositif de chargement selon l'invention permet, par une simple action mécanique de poussée, de plier progressivement la lentille intraoculaire souple initialement à l'état non plié et de l'introduire directement à l'état plié dans une cartouche d'injection, le pliage de la lentille ne nécessitant aucune manipulation manuelle particulière de la cartouche. Le dispositif de chargement selon l'invention sert avantageusement de support de conditionnement de la lentille.

Selon une particularité, le dispositif de chargement comprend des moyens de freinage, ou moyens de retenue, créant au moins une résistance au retour en arrière de la tige du poussoir dans ledit passage interne, ledit tronçon d'extrémité étant apte à recevoir amovible des moyens de maintien s'étendant dans le passage interne jusqu'à son tronçon de réception pour bloquer longitudinalement une lentille disposée dans ce dernier contre l'extrémité libre de la tige du poussoir. Avantageusement, lesdits moyens de freinage créent un blocage au retour en arrière de la tige du poussoir dans le passage interne et, éventuellement une résistance à l'avancement de la tige du poussoir dans le passage interne.

Selon un mode de réalisation, le passage interne de l'élément tubulaire comprend une extrémité distale ouverte par laquelle une cartouche d'injection est emboîtée dans le tronçon d'extrémité de forme cylindrique, l'élément tubulaire étant avantageusement muni d'une fente longitudinale débouchant sur ledit tronçon d'extrémité et s'étendant jusqu'à ladite extrémité distale pour permettre le passage de l'ailette d'une cartouche d'injection. Avantageusement, la fente longitudinale est de type baïonnette, de manière à permettre le blocage d'une cartouche dans le tronçon d'extrémité par enclenchement de son ailette dans ladite fente longitudinale.

Selon un mode de réalisation, le passage interne de l'élément tubulaire comprend une extrémité proximale ouverte par laquelle l'élément support est emboîté dans le tronçon de réception, l'élément support pouvant être formé d'une pointe avec une tête venant s'emboîter dans le tronçon de réception de forme cylindrique, la pointe étant munie d'une fente longitudinale définissant une branche supérieure et une branche inférieure entre lesquelles une lentille peut être reçue, ladite tête de la pointe présentant un canal débouchant entre lesdites branches et dans lequel la tige du poussoir est montée mobile. Ainsi, la lentille est calée radialement entre les deux branches de la pointe et la paroi cylindrique du tronçon de réception.

Le dispositif de chargement comprend avantageusement des moyens de blocage en rotation et en translation de l'élément support dans l'élément tubulaire. Selon un mode de réalisation, la tête de la pointe présente, sur sa paroi cylindrique externe de plus grand diamètre que la pointe, un méplat et un collet qui coopèrent respectivement avec un plat et une gorge annulaire ménagés sur la paroi interne d'une première partie de grand diamètre du tronçon de réception.

Les moyens de freinage de la tige du poussoir comprennent par exemple, des crans transversaux, par exemple formés sur un méplat de la tige, coopérant avec une nervure formée dans le canal de la tête, par exemple sur un plat dudit canal.

Les moyens de maintien peuvent comprendre un embout comportant une tige apte à venir s'emboîter dans le passage interne par l'extrémité distale ouverte dudit passage interne, et à venir s'intercaler entre les deux branches de l'élément support pour bloquer longitudinalement dans le tronçon de réception une lentille entre l'extrémité libre de sa tige et l'extrémité libre de la tige du poussoir. Lesdites tiges peuvent comprendre, à proximité de leur extrémité libre, des renfoncements destinés à recevoir la partie haptique d'une lentille intraoculaire.

Avantageusement, ladite pointe présente une extrémité conique s'étendant dans le tronçon tronconique de pliage du passage interne, les faces externes des parties coniques d'extrémité correspondantes des branches épousant la paroi tronconique du tronçon de pliage, les faces internes opposées desdites parties coniques d'extrémité étant conformées de manière à former des surfaces de guidage pour le pliage de la lentille. Selon un mode de réalisation, la fente de la pointe est décalée par rapport à l'axe longitudinal de la pointe, la partie conique d'extrémité de la branche inférieure présente une face interne concave, la partie conique d'extrémité de la branche supérieure présentant une face interne convexe. A titre d'exemple, les parties coniques d'extrémité des branches s'étendent dans le tronçon d'extrémité de l'élément tubulaire et sont aptes à venir s'insérer dans la section tronconique d'entrée de la chambre de réception d'une cartouche d'injection placée dans ledit tronçon d'extrémité.

Selon une particularité, l'élément tubulaire comprend des orifices latéraux débouchant dans le tronçon de réception, entre les branches de l'élément tubulaire pour permettre le passage d'agent de stérilisation, de lubrifiants et/ou d'un outil pour le positionnement de la partie haptique de la lentille par rapport à la tige de l'embout de maintien.

Le dispositif comprend avantageusement des surfaces d'appui pour l'actionnement du poussoir, formées par un bouton de manoeuvre à l'extrémité proximale de la tige du poussoir et de deux pattes diamétralement opposées sur la paroi externe de l'élément tubulaire, le dispositif de chargement se présentant ainsi sous la forme d'un dispositif de type seringue.

La présente invention propose en outre un dispositif de chargement, tel que décrit précédemment, chargé d'une lentille intraoculaire souple et emballé dans un étui stérile.

L'invention a également pour objet une cartouche d'injection destinée à être chargée d'une lentille intraoculaire souple au moyen du dispositif de chargement décrit ci-dessus, comprenant une chambre de réception d'une lentille sous forme pliée prolongée par une canule d'injection pour l'injection de la lentille pliée, caractérisée en ce que la chambre de réception est formée d'un tube unique et présente une section tronconique d'entrée de la chambre de réception apte a recevoir les parties coniques d'extrémité des branches s'étendent dans le tronçon d'extrémité de l'élément tubulaire. Le dispositif de chargement selon l'invention, utilisant une simple action de poussée pour le pliage et le chargement de la lentille, permet d'utiliser des cartouches d'injection sans partie mobile, simples à produire.

Selon une particularité, la chambre de réception comprend une section tronconique délimitée d'un côté par l'orifice d'entrée de la chambre de réception et se prolongeant de l'autre côté par une section cylindrique, ladite section tronconique, destinée à recevoir les parties coniques d'extrémité de l'élément support, permettant de réduire la longueur du dispositif de chargement. Avantageusement, ledit tube comprend sur sa paroi externe une ailette longitudinale rectangulaire s'étendant radialement vers l'extérieur, permettant son blocage sur le dispositif de chargement.

L'invention a également pour objet un ensemble comprenant un dispositif de chargement, tel que décrit ci-dessus, et une cartouche d'injection, notamment une cartouche selon l'invention tel que défini ci-dessus.

L'invention sera mieux comprise, et d'autres buts, détails, caractéristiques et avantages apparaîtront plus clairement au cours de la description explicative détaillée qui va suivre d'un mode de réalisation particulier actuellement préféré de l'invention, en référence aux dessins schématiques annexés sur lesquels :
- la figure 1 représente une vue en perspective d'un dispositif de chargement selon l'invention, équipé d'un embout de maintien pour le stockage et le transport d'une lentille avant utilisation;
- la figure 2 représente une vue en perspective du dispositif de la figure 1, équipé d'une cartouche d'injection;
- la figure 3 représente une vue en coupe longitudinale selon le plan III-III du dispositif de la figure 1 ;
- la figure 4 représente une vue en coupe longitudinale selon le plan IV-IV de la figure 1, illustrant la branche inférieure de l'élément support ;
- la figure 5 représente une vue en coupe longitudinale selon le plan V-V du dispositif de la figure 2 ;
- la figure 6 représente une vue en coupe longitudinale selon le plan VI-VI de la figure 2 illustrant la branche inférieure de l'élément support ;
- les figures 7 et 8 représentent respectivement des vues en coupe transversale selon les plans de coupe VII-VII et VIII-VIII de la figure 5 ;
- la figure 9 représente une vue en perspective de l'élément support du dispositif des figures 1 à 6 ;
- la figure 10 représente une vue en perspective de la tige de poussoir du dispositif des figures 1 à 6 ;
- la figure 11 représente une vue en perspective de l'embout de maintien du dispositif des figures 1, 3 et 4 ;
- la figure 12 est une vue en perspective de la cartouche des figures 2, 5 et 6 ;
- lès figures 13 et 14 sont des vues en coupe analogue à celles des figures 3 et 4, illustrant un dispositif selon une variante de réalisation équipé d'un embout ; et,
- les figures 15 et 16 sont des vues en coupe analogues à celles des figures 5 et 6, illustrant le dispositif des figures 13 et 14 équipé d'une cartouche d'injection.

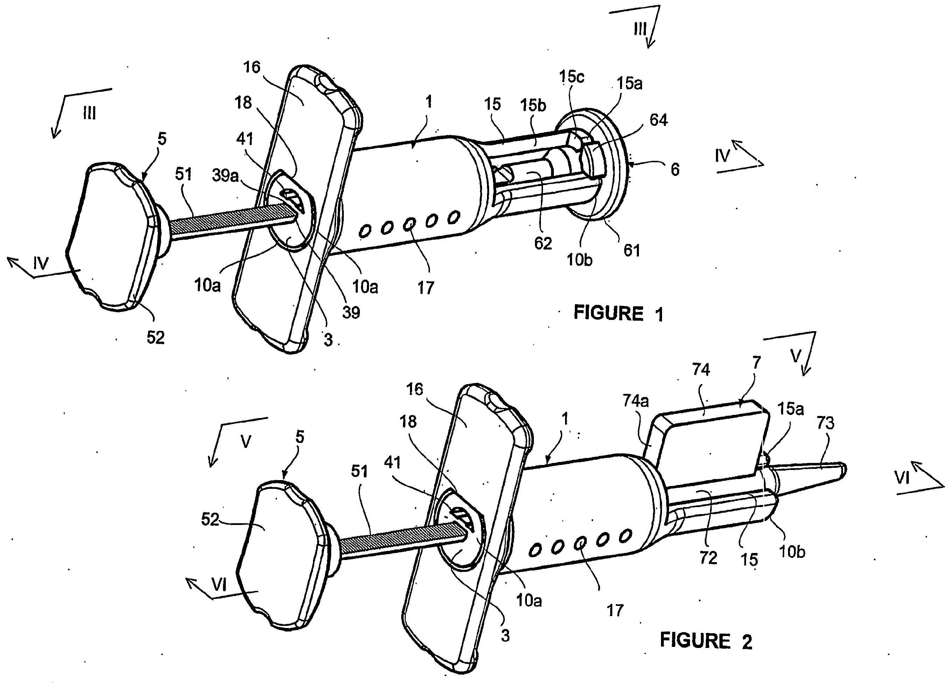

Le dispositif de chargement représenté sur les figures 1 à 6 se présente sous la forme d'un dispositif de type seringue. Il comporte un élément tubulaire 1 comprenant un passage interne 10 d'axe longitudinal A, dans lequel sont insérés par son extrémité proximale ouverte 10a un élément support de lentille 3 et la tige 51 d'un poussoir 5, et par son extrémité distale ouverte 10b un embout de maintien amovible 6 ou une cartouche d'injection 7.

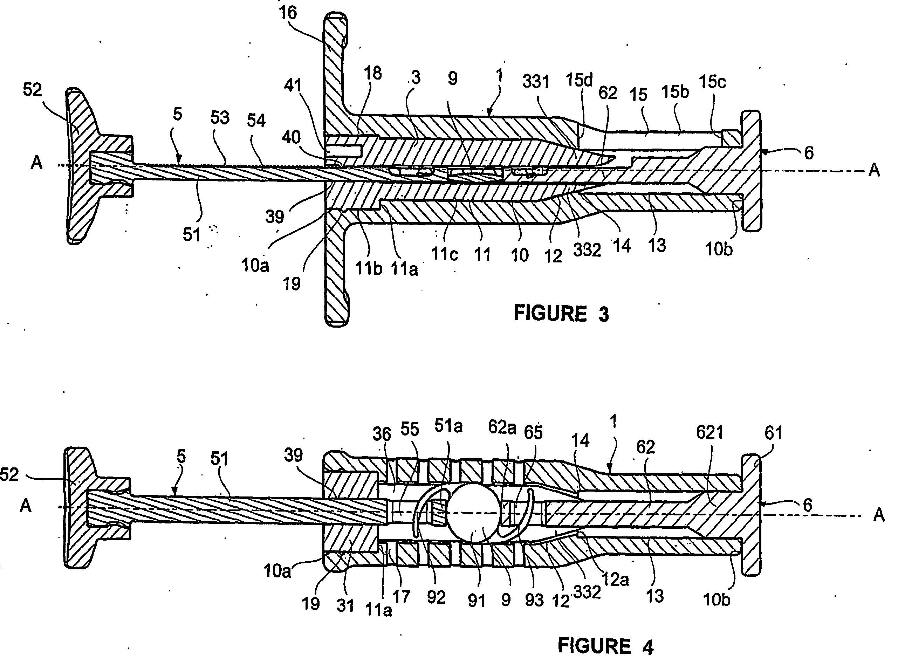

En référence aux figures 3 et 4, le passage interne 10 comprend, de son extrémité proximale 10a à son extrémité distale 10b, un premier tronçon cylindrique 11, dit de réception, pour recevoir une lentille 9 à l'état déployé ou non plié, se prolongeant par un deuxième tronçon tronconique 12, dit de pliage, dont le diamètre interne se réduit en direction de l'extrémité distale jusqu'à un orifice de sortie 12a de petit diamètre, ce tronçon de pliage débouchant sur un troisième tronçon cylindrique d'extrémité 13, destiné à recevoir une cartouche d'injection. Le diamètre de ce tronçon d'extrémité est supérieur au diamètre de l'orifice de sortie 12a, le tronçon de pliage et le tronçon d'extrémité définissant ainsi un épaulement 14. La paroi interne du tronçon de réception présente un épaulement 11a orienté vers l'extrémité proximale 10a, séparant le tronçon de réception en une première partie 11b de grand diamètre et une seconde partie 11c de petit diamètre.

L'élément tubulaire présente une fente longitudinale 15, de type baïonnette, pour l'assemblage d'un embout de maintien, appelé également bouchon de maintien, ou d'une cartouche tel que décrit ci-après. Suivant les figures 1 à 3, la fente 15 comprend une première partie 15a qui part de l'extrémité distale 10b et qui se prolonge par une deuxième partie 15b, cette deuxième partie s'étendant sur un secteur angulaire plus grand que la première partie pour définir une butée de retenue 15c. Cette fente s'étend sur toute la longueur du tronçon d'extrémité 13, ainsi que sur une partie du tronçon de pliage, effaçant ainsi l'épaulement 14 sur un secteur correspondant à celui de la deuxième partie de la fente. Deux pattes 16 diamétralement opposées, s'étendant radialement vers l'extérieur, sont prévues sur la paroi externe de l'élément tubulaire, du côté de son extrémité proximale 10a, pour former des surfaces d'appui pour actionner le poussoir.

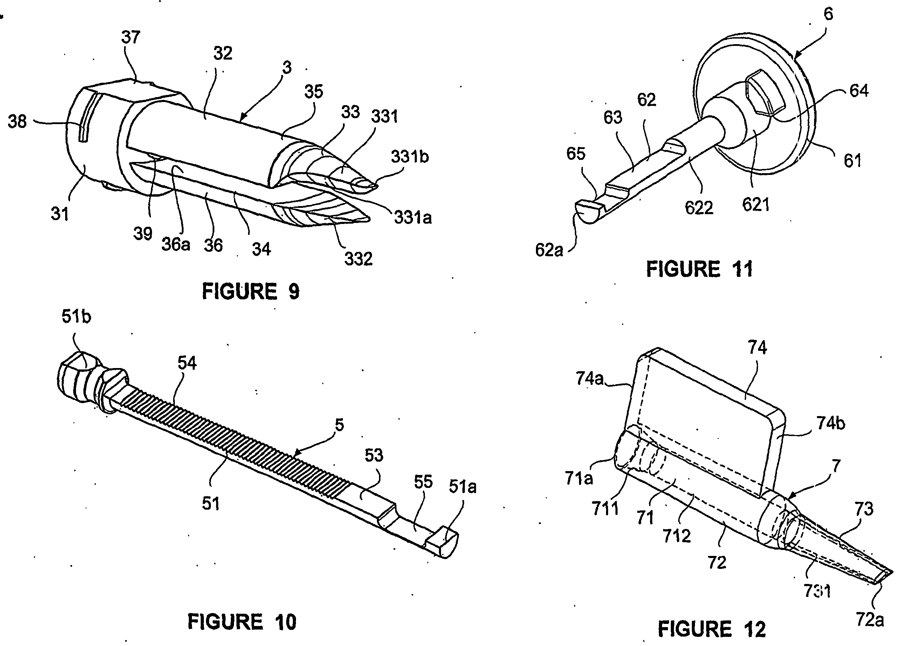

Suivant la figure 9, l'élément support 3, destiné à supporter une lentille dans un état non plié, est formé d'une pointe ou poinçon fendu, comprenant une tête cylindrique et une extrémité conique, dont la forme extérieure épouse le tronçon de réception et le tronçon de pliage. En référence à la figure 9, l'élément support 3 comprend une tête ou embase cylindrique 31 dont le diamètre extérieur correspond sensiblement au diamètre interne de la première partie 11b du tronçon de réception, et une pointe comprenant une partie cylindrique 32 dont le diamètre correspond sensiblement au diamètre interne de la deuxième partie 11c, se prolongeant par une partie conique 33. Une fente longitudinale 34, décalée par rapport à l'axe longitudinal de l'élément support s'étend sur toute la longueur de la pointe pour définir une branche dite supérieure 35 et une branche dite inférieure 36.

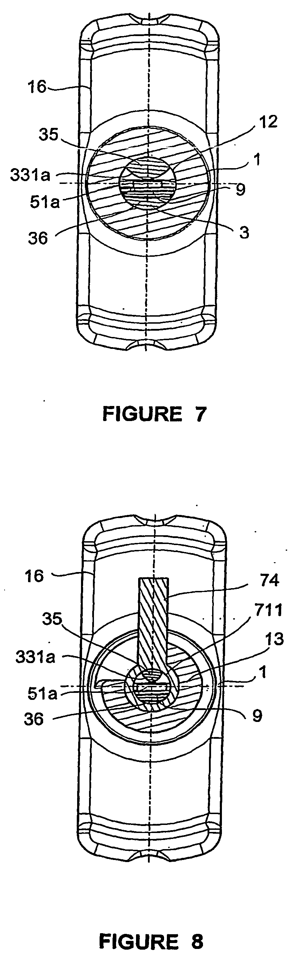

En référence aux figures 3, 4 et 7, lorsque l'élément support est inséré dans l'élément tubulaire, les parties coniques 331, 332 des branches viennent en contact par leur face extérieure contre la paroi tronconique du tronçon de pliage et s'étendent au-delà de l'orifice de sortie 12a dans le tronçon d'extrémité 13. La face 36a de la branche inférieure, opposée à la branche supérieure, sur laquelle la lentille sera disposée, est légèrement concave sur toute sa longueur. Les arêtes latérales de la partie conique 331 de la branche supérieure 35 sont adoucies pour former une face convexe de guidage 331 a opposée à la branche inférieure pour guider le pliage de la lentille. La convexité de cette face de guidage se relève symétriquement jusqu'à l'extrémité 331b de la branche supérieure, dans la direction opposée à la branche inférieure.

Le blocage en rotation de l'élément support dans l'élément tubulaire est réalisé par un méplat 37 formé sur la paroi externe de l'embase 31 qui coopère avec un plat 18 ménagé sur la paroi interne de la première partie 11b du tronçon de réception. Un collet 38 (figure 9), par exemple discontinu, est prévu sur la paroi cylindrique externe de l'embase pour venir se loger dans une gorge annulaire 19 correspondante de la première partie 11b afin d'assurer le blocage en translation de l'élément support dans l'élément tubulaire. Des orifices latéraux 17, disposés en deux ensembles diamétralement opposés, sont ménagés dans la paroi de l'élément tubulaire, ces orifices débouchant dans le tronçon de réception, entre les deux branches de l'élément support.

En référence en particulier aux figures 3 et 10, le poussoir 5 comprend une tige cylindrique 51 équipé à son extrémité proximale 51b de plus grande section d'un bouton de manoeuvre 52 pour son actionnement. La tige présente un méplat 53 et est apte à venir s'insérer dans un canal 39 de l'embase débouchant entre les deux branches. Le méplat comprend des stries transversales 54 coopérant avec une nervure 40 formée sur le plat 39a (figure 1) du canal 39 de l'embase. L'embase comprend un évidement d'affaiblissement 41, par exemple de section globalement semi-circulaire, qui s'étend parallèlement au-dessus du plat, de sorte que la nervure puisse se déformer élastiquement et ainsi permettre le déplacement du poussoir vers l'intérieur.

Suivant la figure 11, l'embout de maintien 6 comprend un disque 61 portant une tige axiale 62 apte à venir s'insérer dans l'élément tubulaire par son extrémité distale pour venir bloquer longitudinalement, contre l'extrémité libre de la tige du poussoir, la lentille disposée entre les deux branches. La tige comprend une première partie 621 dont le diamètre correspond à celle du tronçon d'extrémité 13 pour assurer le calage axial de l'embout sur l'élément tubulaire, qui se prolonge par une deuxième partie 622 de diamètre inférieur au diamètre de l'orifice de sortie 12a. Cette deuxième partie présente un méplat 63 pour permettre son insertion entre les deux branches, avec sa paroi cylindrique contre la face concave 36a de branche inférieure, et son méplat en vis-à-vis de la branche supérieure 35. Le blocage en rotation de l'embout est assuré par un plot 64 en secteur circulaire prévu sur la première partie de la tige venant se loger dans la première partie 15a de la fente. Le maintien de l'embout sur l'élément tubulaire est assuré par l'emboîtement de la première partie 621 de la tige et du plot respectivement dans le passage interne 10 et dans la fente 15.

Pour la réception d'une lentille 9 comportant une partie optique 91 et une partie haptique constituée par exemple de deux anses latérales 92, 93, tel qu'illustré à la figure 4, le méplat 53 de la tige 51 du poussoir présente à proximité de l'extrémité libre 51a un renfoncement transversal 55 destinée à recevoir l'une des anses, le méplat 63 de la tige 62 de l'embout présentant à proximité de l'extrémité libre 62a un renfoncement transversal 65 pour recevoir l'autre anse.

La figure 12 représente une cartouche d'injection monobloc, sans ailettes mobiles, utilisable avec le dispositif de chargement décrit ci-dessus. La cartouche comprend une chambre de réception 71 de la lentille sous forme pliée, formée d'un tube unique 72, prolongée par une canule d'injection 73 pour l'injection de la lentille pliée. La chambre de réception comprend une section tronconique 711 délimitée d'un côté par l'orifice d'entrée 71 a de la chambre de réception et se prolongeant de l'autre côté par une section cylindrique 712. La canule d'injection 73 comprend un canal interne 731 disposé dans le prolongement de la section cylindrique 712 et présentant un diamètre interne se réduisant légèrement en direction de l'extrémité distale 72a de la canule. L'extrémité distale est avantageusement biseautée pour faciliter l'insertion de la canule dans l'oeil. Le tube comprend sur sa paroi externe une ailette longitudinale rectangulaire 74 s'étendant radialement vers l'extérieur.

Une description de l'utilisation du dispositif selon invention va à présent être effectuée.

Dans un premier temps, la tige 51 du poussoir 5 est insérée dans le canal 39 de l'élément support jusqu'à ce que son extrémité libre 51a soit disposée entre les deux branches 35, 36, tel qu'illustré aux figures 3 et 4. Une lentille intraoculaire 9, comprenant par exemple une partie optique 91 et deux anses 92, 93, est disposée entre les deux branches de l'élément support, sur la face concave de la branche inférieure, contre l'extrémité libre 51a de la tige du poussoir, l'une 92 de ses anses étant amenée dans le renfoncement 55 de la tige. L'élément support est alors inséré dans l'élément tubulaire. Son embase 31 vient en butée contre l'épaulement 11a et le collet 38 vient se clipser dans la gorge annulaire 19. L'embout de maintien 6, tenu par son disque 61, est ensuite inséré dans l'élément tubulaire par l'extrémité distale. Son disque vient en butée contre l'extrémité distale et l'extrémité libre de la tige vient contre la partie optique 91 de la lentille. Avant l'insertion complète de l'embout ou une fois cette insertion effectuée, la deuxième anse 93 de la lentille est amenée dans le renfoncement 65 de la tige de l'embout au moyen d'un outil approprié, tel qu'une micro-pince, introduit par l'un des orifices latéraux 17.

Après une opération de stérilisation, au moyen d'un agent de stérilisation passant par les orifices latéraux, le dispositif ainsi pré-chargé avec une lentille peut être placé dans un étui stérile pour son stockage, son transport et sa commercialisation. La lentille à l'état non plié est maintenue radialement entre les deux branches et la paroi cylindrique de la deuxième partie 11c du tronçon de réception et est maintenue longitudinalement entre les extrémités libres de la tige de l'embout amovible et de la tige du poussoir. Les crans 54 sur la tige du poussoir coopérant avec la nervure 40 permettent un positionnement précis du poussoir par rapport à l'embout pour un calage longitudinal optimal de la lentille.

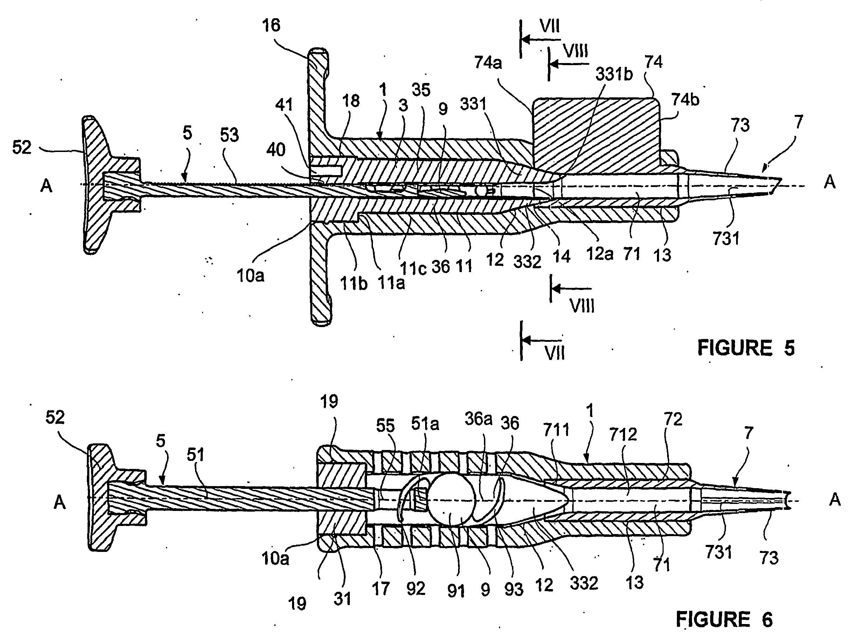

Pour le chargement de la lentille dans une cartouche d'injection, l'embout est retiré de l'élément tubulaire et est remplacé par une cartouche d'injection. Lors du retrait de l'embout amovible, la deuxième anse placée dans le renfoncement de la lentille est tirée vers l'avant. La cartouche est insérée dans l'élément tubulaire par la fente jusqu'à ce que l'extrémité du tube 72 définissant l'orifice d'entrée 71a de la chambre de réception vienne en butée contre l'épaulement 14, et l'ailette en butée par son bord arrière 74a contre l'extrémité arrière 15d (figure 3) de la fente. On fait alors pivoter la cartouche d'environ 45° pour enclencher l'ailette dans la deuxième partie 15b de la fente, son bord 74b avant contre la butée de retenue 15c. Dans cette position de la cartouche, les parties coniques 331, 332 de la branche inférieure et de la branche supérieure s'étendent dans la section tronconique 711 de la chambre de réception. Tel que visible sur les figures 5, 6 et 8, la partie inférieure de la paroi cylindrique définissant la section cylindrique 712 de la chambre de réception est disposée dans le prolongement de la face concave de la branche inférieure. Tel que visible sur la figure 5, l'extrémité 331b de la branche supérieure vient se positionner dans la section cylindrique 712 de la chambre, sa face extérieure contre la paroi de cette section cylindrique.

Le praticien peut alors introduire mécaniquement la lentille dans la cartouche d'injection en actionnant le poussoir. Lors de l'avancée du poussoir, la lentille est poussée par l'extrémité libre du poussoir dans la chambre de réception. La lentille qui se déplace le long de la face concave de la branche inférieure vient latéralement en appui contre la paroi tronconique du tronçon de pliage et s'enroule progressivement sur elle-même pour venir se loger dans la chambre de réception, la face convexe symétrique de guidage permet de guider cet enroulement progressif en assurant le maintien de la lentille contre la face concave de la branche inférieure lors de l'enroulement ou pliage. La cartouche ainsi chargée de sa lentille peut être retirée du dispositif de chargement et être positionnée dans un injecteur classique pour son insertion dans l'oeil par une petite incision. Le praticien pourra introduire des lubrifiants, tels qu'une solution saline ou un produit viscoélastique à l'intérieur du dispositif par les orifices latéraux 17 et/ou à l'intérieur de la cartouche avant sa mise en place dans le dispositif, afin de faciliter l'action de poussée et l'opération de pliage de la lentille dans sa cartouche d'injection. Avantageusement, les moyens de retenue de la tige du poussoir dans le canal de l'embase, constitués par les crans 54 et la nervure 40, sont conformés de manière à former un système anti-retour interdisant le retrait du poussoir hors de l'élément tubulaire, et donc toute réutilisation du dispositif de chargement.

Les dimensions du dispositif de chargement selon l'invention, en particulier les dimensions de son passage interne et de son élément support, ainsi que la longueur de la tige de l'embout de maintien, seront adaptées pour un type de lentille donné, notamment en fonction de la taille de sa partie optique, ainsi que la taille, la forme et la disposition de son éventuelle partie haptique, pour permettre le calage longitudinal et radial puis le pliage progressif de la lentille. Des dispositifs de chargement présentant des dimensions différentes pourront ainsi être proposés pour les différents types de lentille présents sur le marché. Par ailleurs, la convexité de la face de guidage 331a sera adaptée aux différents types de lentilles, celle-ci pouvant être symétrique ou asymétrique, continue ou évolutive.

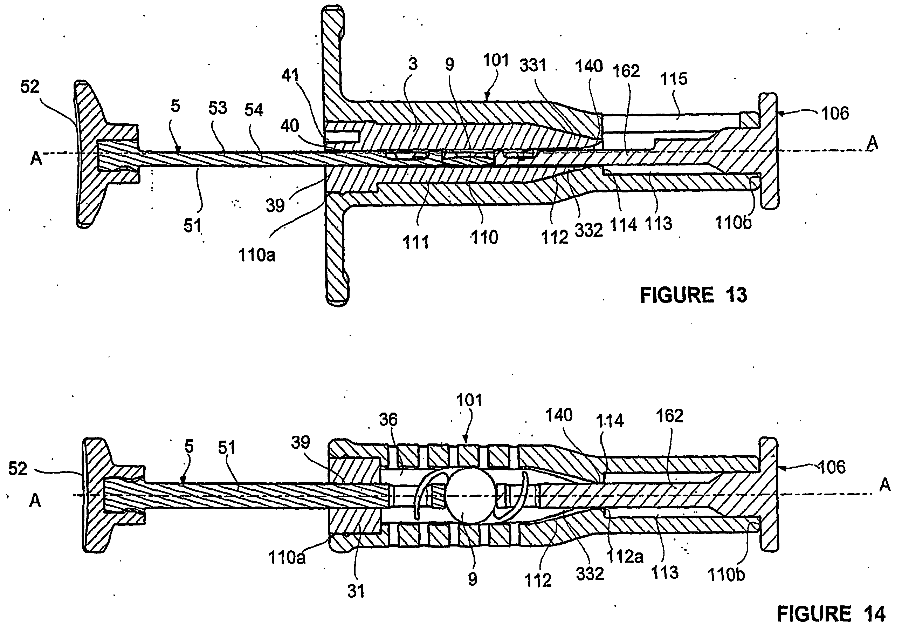

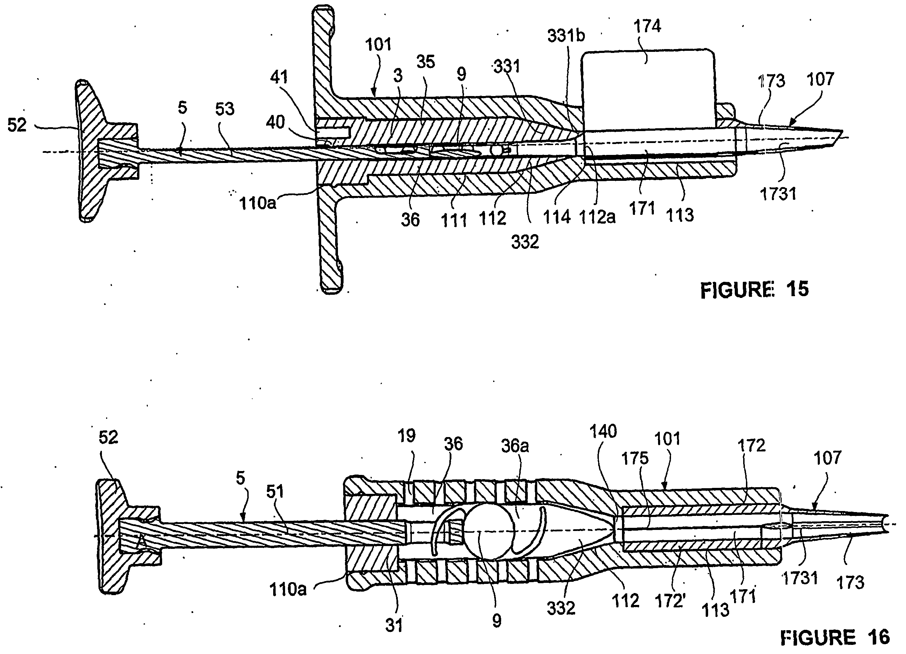

Les figures 13 à 16 illustrent un dispositif de chargement selon une variante de réalisation, utilisable avec une cartouche classique à ailettes mobiles. En référence aux figures 15 et 16, une cartouche d'injection, dite classique 107, comprend une chambre de réception cylindrique 171 se prolongeant par une canule d'injection 173, la chambre de réception étant formée de deux demi-tubes 172, 172' de section semi-circulaire, reliés par une ligne de liaison formant charnière 175, chaque tube étant muni d'une ailette 174 pour permettre la fermeture et l'ouverture de la chambre.

Dans cette variante, les parties coniques 331, 332 de l'élément support ne s'étendent pas dans la chambre de réception de la cartouche. L'élément support 3 et le poussoir 5 sont identiques à ceux décrit précédemment. Le dispositif diffère de celui décrit précédemment par le fait que le passage interne 110 de l'élément tubulaire 101 présente un deuxième tronçon tronconique de pliage 112 de plus grande longueur pour recevoir l'intégralité des parties coniques 331, 332 des deux branches, à l'exception de l'extrémité recourbée 331b de la branche supérieure, le tronçon tronconique se prolongeant par un tronçon intermédiaire cylindrique 140 dont l'orifice de sortie 112a débouche sur le tronçon d'extrémité 113 et dans lequel vient se placer ladite extrémité recourbée. La fente 115 s'étend de l'extrémité distale 110b du passage interne jusqu'à l'épaulement 114. Par ailleurs, l'embout 106 présente également une tige 162 de longueur légèrement supérieure à celle décrite précédemment, venant se loger dans le tronçon de réception 111 qui s'étend de l'extrémité proximale 110a au tronçon de pliage.

La mise en place de la cartouche sur le dispositif s'effectue après avoir ramener les deux ailettes l'une vers l'autre pour fermer la chambre d'injection. Après chargement de la lentille sous forme pliée dans la chambre de réception, la cartouche est retirée du dispositif en maintenant la chambre fermée, puis est chargée dans un injecteur classique.

Bien que l'invention ait été décrite en liaison avec un mode de réalisation particulier, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Dispositif de chargement d'une lentille intraoculaire souple dans une cartouche d'injection comprenant une chambre de réception d'une lentille et une canule d'injection, comprenant
- un élément tubulaire (1, 101) ayant un passage interne (10, 110), ledit passage comprenant un tronçon de réception (11, 111) d'une lentille (9) à l'état non plié, un tronçon intermédiaire tronconique de pliage (12, 112), et un tronçon d'extrémité (13, 113) apte à recevoir amovible une cartouche d'injection (7, 107), d'une manière telle que le tronçon intermédiaire tronconique de pliage débouche sur la chambre de réception (71, 171) de la cartouche d'injection,
- un élément support (3) monté dans ledit tronçon de réception apte à supporter une lentille dans un état non plié, et
- un poussoir (5) comprenant une tige (51) montée mobile dans ledit passage interne, apte à pousser une lentille (9) disposée dans le tronçon de réception à travers le tronçon intermédiaire tronconique de pliage pour plier ladite lentille progressivement, puis dans la chambre de réception d'une cartouche d'injection placée dans le tronçon d'extrémité.

2. Dispositif selon la revendication 1, **caractérisé en ce qu'**il comprend des moyens de freinage (54, 40) créant au moins une résistance au retour en arrière de la tige (51) du poussoir dans ledit passage interne (10, 110), ledit tronçon d'extrémité (13, 113) étant apte à recevoir amovible des moyens de maintien (6, 106) s'étendant dans le passage interne jusqu'à son tronçon de réception pour bloquer longitudinalement une lentille (9) disposée dans ce dernier contre l'extrémité libre (51 a) de la tige du poussoir.

3. Dispositif selon la revendication 2, **caractérisé en ce que** lesdits moyens de freinage créent un blocage au retour en arrière de la tige (51) du poussoir (5) dans le passage interne (10, 110) et une résistance à l'avancement de la tige (51) du poussoir dans le passage interne.

4. Dispositif selon l'une des revendications 1 à 3, **caractérisé en ce que** le passage interne (10, 110) de l'élément tubulaire (1, 101) comprend une extrémité distale ouverte (10b, 110b) par laquelle une cartouche d'injection (7, 107) est emboîtée dans le tronçon d'extrémité (13, 113) de forme cylindrique, l'élément tubulaire étant muni d'une fente longitudinale (15, 115) débouchant sur ledit tronçon d'extrémité et s'étendant jusqu'à ladite extrémité distale pour permettre le passage de l'ailette (74, 174) d'une cartouche d'injection.

5. Dispositif selon la revendication 4, **caractérisé en ce que** la fente longitudinale (15, 115) est de type baïonnette, de manière à permettre le blocage d'une cartouche d'injection (7, 107) dans le tronçon d'extrémité (13, 113) par enclenchement de son ailette (74,174) dans ladite fente longitudinale.

6. Dispositif selon l'une des revendications 1 à 5, **caractérisé en ce que** le passage interne (10, 110) de l'élément tubulaire comprend une extrémité proximale (10a, 110a) ouverte par laquelle l'élément support (3) est emboîté dans le tronçon de réception (11, 111), et **en ce que** l'élément support (3) est formé d'une pointe (32, 33) avec une tête (31) venant s'emboîter dans le tronçon de réception de forme cylindrique (11, 111), la pointe étant munie d'une fente longitudinale (34) définissant une branche supérieure (35) et une branche inférieure (36) entre lesquelles une lentille (9) peut être reçue, ladite tête de la pointe présentant un canal (39) débouchant entre lesdites branches et dans lequel la tige (51) du poussoir est montée mobile.

7. Dispositif selon les revendications 2 et 6, **caractérisé en ce que** les moyens de maintien comprennent un embout (6, 106) comprenant une tige (62) apte à venir s'emboîter dans le passage interne (10, 110) par son extrémité distale ouverte (10b, 110b) et à venir s'intercaler entre les deux branches (35, 36) de l'élément support pour bloquer longitudinalement dans le tronçon de réception (11, 111) une lentille entre l'extrémité libre (51 a) de sa tige et l'extrémité libre (62a) de la tige du poussoir.

8. Dispositif selon la revendication 6 ou 7, **caractérisé en ce que** ladite pointe (32, 33) présente une extrémité conique s'étendant dans le tronçon tronconique de pliage (12, 112) du passage interne, les faces externes des parties coniques d'extrémité correspondantes (331, 332) des branches (35, 36) épousant la paroi tronconique du tronçon de pliage, les faces internes opposées (36a, 331 a) desdites parties coniques d'extrémité étant conformées de manière à former des surfaces de guidage pour le pliage de la lentille.

9. Dispositif selon la revendication 8, **caractérisé en ce que** la fente (34) de la pointe est décalée par rapport à l'axe longitudinal de la pointe, la partie conique d'extrémité (332) de la branche inférieure (36) présente une face interne concave (36a), la partie conique d'extrémité (331) de la branche supérieure (35) présentant une face interne convexe (331 a).

10. Dispositif selon la revendication 8 ou 9, **caractérisé en ce que** les parties coniques d'extrémité (331, 332) des branches s'étendent dans le tronçon d'extrémité (13) de l'élément tubulaire et sont aptes à venir s'insérer dans la section tronconique (711) d'entrée de la chambre de réception (71) d'une cartouche d'injection (7) placée dans ledit tronçon d'extrémité (13).

11. Dispositif selon l'une dés revendications 6 à 10, **caractérisé en ce que** l'élément tubulaire (1, 101) comprend des orifices latéraux (17) débouchant dans le tronçon de réception (11, 111), entre les branches (35,36) de l'élément tubulaire, pour permettre le passage d'agent de stérilisation, de lubrifiants et/ou d'outil pour le positionnement de la partie haptique de la lentille par rapport à la tige de l'embout de maintien.

12. Dispositif selon l'une des revendications 1 à 11, **caractérisé en ce qu'**il est chargé d'une lentille intraoculaire souple (9) et emballé dans un étui stérile.

13. Cartouche d'injection destinée à être chargée d'une lentille intraoculaire souple au moyen du dispositif de chargement selon l'une des revendications 1 à 11, comprenant une chambre de réception d'une lentille sous forme pliée prolongée par une canule d'injection pour l'injection de la lentille pliée, **caractérisée en ce que** la chambre de réception (71) est formée d'un tube unique (72) et présente une section tronconique (711) d'entrée de la chambre de réception (71) apte à recevoir les parties coniques d'extrémité (331, 332) des branches s'étendant dans le tronçon d'extrémité (13) de l'élément tubulaire.

14. Cartouche selon la revendication 13, **caractérisée en ce que** la chambre de réception (71) comprend une section tronconique (711) délimitée d'un côté par l'orifice d'entrée (71 a) de la chambre de réception et se prolongeant de l'autre côté par une section cylindrique (712).

15. Cartouche selon la revendication 13 ou 14, **caractérisée en ce que** ledit tube comprend sur sa paroi externe une ailette longitudinale (74) s'étendant radialement vers l'extérieur.

16. Ensemble comprenant un dispositif de chargement selon l'une des revendications 1 à 12 et une cartouche d'injection (7, 107) comportant une chambre de réception (71, 171) et une canule d'injection (73, 173).

17. Ensemble selon la revendication 16, **caractérisé en ce qu'**il comprend une cartouche d'injection (7) selon l'une des revendications 13 à 15.

## Patentansprüche

1. Vorrichtung zum Laden einer weichen Intraokularlinse in eine Injektionskartusche, die eine Kammer zum Aufnehmen einer Linse und eine Injektionskanüle umfasst, umfassend
- ein röhrenförmiges Element (1, 101) mit einem inneren Durchlass (10, 110), wobei dieser Durchlass ein Aufnahmeteilstück (11, 111) für eine Linse (9) in einem nicht gefalteten Zustand, ein kegelstumpfförmiges Zwischenteilstück zum Falten (12, 112) sowie ein Endteilstück (13, 113) umfasst, das eine Injektionskartusche (7, 107) herausnehmbar auf eine Weise aufnehmen kann, sodass das kegelstumpfförmige Zwischenteilstück zum Falten in der Aufnahmekammer (71, 171) der Injektionskartusche mündet,
- ein Stützelement (3), das in diesem Aufnahmeteilstück angebracht ist und und eine Linse in einem nicht gefalteten Zustand tragen kann, und
- einen Schieber (5), umfassend einen Schaft (51), der beweglich in diesem inneren Durchlass angebracht ist und eine in dem Aufnahmeteilstück angeordnete Linse (9) zum allmählichen Falten der Linse durch das kegelstumpfförmige Zwischenteilstück zum Falten und dann in die Aufnahmekammer einer Injektionskartusche schieben kann, die in dem Endteilstück platziert wurde.

2. Vorrichtung gemäß Anspruch 1, **dadurch gekennzeichnet, dass** sie Bremsmittel (54, 40) umfasst, die mindestens einen Widerstand gegenüber einem Zurückkehren des Schafts (51) des Schiebers in diesem inneren Durchgang (10, 110) schaffen, wobei das Endteilstück (13, 113) herausnehmbar Mittel zum Halten (6, 106) aufnehmen kann, die sich in dem inneren Durchlass bis zum Aufnahmeteilstück erstrecken, um eine in letzterem angeordnete Linse (9) gegenüber dem freien Ende (51a) des Schafts des Schiebers zu blockieren.

3. Vorrichtung gemäß Anspruch 2, **dadurch gekennzeichnet, dass** diese Bremsmittel eine Blockierung des Zurückkehrens des Schafts (51) des Schiebers (5) in dem inneren Durchlass (10, 110) und einen Widerstand gegenüber dem Vorbewegen des Schafts (51) des Schiebers in dem inneren Durchlass schaffen.

4. Vorrichtung gemäß einem der Ansprüche 1 bis 3, **dadurch gekennzeichnet, dass** der innere Durchlass (10, 110) des röhrenförmigen Elements (1, 101) ein offenes distales Ende (10b, 110b) umfasst, durch das eine Injektionskartusche (7, 107) in das Endteilstück (13, 113) der zylindrischen Form eingepasst wird, wobei das röhrenförmige Element mit einem Längsschlitz (15, 115) versehen ist, der in diesem Endteilstück mündet und sich bis zu diesem distalen Ende erstreckt, um den Durchgang des Flügels (74, 174) einer Injektionskartusche zu gestatten.

5. Vorrichtung gemäß Anspruch 4, **dadurch gekennzeichnet, dass** der Längsschlitz (15, 115) vom Bajonetttyp ist, um eine Blockierung einer Injektionskartusche (7, 107) im Endteilstück (13, 113) durch ein Einklinken ihres Flügels (74, 174) in diesen Längsschlitz zu gestatten.

6. Vorrichtung gemäß einem der Ansprüche 1 bis 5, **dadurch gekennzeichnet, dass** der innere Durchlass (10, 110) des röhrenförmigen Elements ein offenes proximales Ende (10a, 110a) umfasst, durch das das Stützelement (3) in das Aufnahmeteilstück (11, 111) eingeführt wird, und dieses Stützelement (3) durch einen Stift (32, 33) mit einem Kopf (31) gebildet wird, der in das Aufnahmeteilstück der zylindrischen Form (11, 111) eingeführt wird, wobei der Stift mit einem Längsschnitt (34) versehen ist, der einen oberen Arm (35) und einen unteren Arm (36) definiert, zwischen denen eine Linse (9) aufgenommen werden kann, wobei der Kopf dieses Stifts einen Kanal (39) aufweist, der zwischen diesen Armen mündet und in dem der Schaft (51) des Schiebers beweglich montiert ist.

7. Vorrichtung gemäß der Ansprüche 2 und 6, **dadurch gekennzeichnet, dass** die Mittel zum Halten ein Ansatzstück (6, 106) umfassen, die einen Schaft (62) umfassen, der durch das offene distale Ende (10b, 110b) des inneren Durchlasses (10, 110) eingeführt werden kann und zwischen die beiden Arme (35, 36) des Stützelements eingeschoben werden kann, um eine Linse zwischen dem freien Ende (51a) seines Schafts und dem freien Ende (62a) des Schafts des Schiebers im Aufnahmeteilstück (11, 111) longitudinal zu blockieren.

8. Vorrichtung gemäß Anspruch 6 oder 7, **dadurch gekennzeichnet, dass** dieser Stift (32, 33) ein konisches Ende aufweist, das sich im kegelstumpfförmigen Zwischenteilstück zum Falten (12,112) des inneren Durchlasses erstreckt, wobei die äußeren Seiten der konischen Teile der korrespondierenden Enden (331, 332) der Arme (35, 36) die kegelstumpfförmige Wandung des Teilstücks zum Falten umschließen, während die gegenüberliegenden Innenseiten (36a, 331a) dieser konischen Endteile angepasst sind, um Führungsoberflächen zum Falten der Linse zu bilden.

9. Vorrichtung gemäß Anspruch 8, **dadurch gekennzeichnet, dass** der Schlitz (34) des Stifts in Bezug auf die Längsachse des Stifts verschoben ist, wobei der konische Endteil (332) des unteren Arms (36) eine konkave Innenseite (36a) aufweist, wobei der konische Endteil (331) des oberen Arms (35) eine konkave Innenseite (331a) aufweist.

10. Vorrichtung gemäß Anspruch 8 oder 9, **dadurch gekennzeichnet, dass** die konischen Endteile (331, 332) der Arme sich im Endteilstück (13) des röhrenförmigen Elements erstrecken und in den kegelstumpfförmigen Eingangsbereich (711) der Aufnahmekammer (71) einer Injektionskartusche (7) eingeschoben werden können, die in diesem Endteilstück (13) platziert wurde.

11. Vorrichtung gemäß einem der Ansprüche 6 bis 10, **dadurch gekennzeichnet, dass** das röhrenförmige Element (1, 101) seitliche Öffnungen (17) umfasst, die in dem Aufnahmeteilstück (11, 111) zwischen den Armen (35, 36) des röhrenförmigen Elements münden, um den Durchgang von Sterilisationsmittel, Gleitmitteln und/oder Werkzeugen zum Positionieren des haptischen Bereichs der Linse in Bezug auf den Schaft des Haltestutzens zu erlauben.

12. Vorrichtung gemäß einem der Ansprüche 1 bis 11, **dadurch gekennzeichnet, dass** sie mit einer weichen Intraokularlinse (9) beladen und in einem sterilen Etui verpackt ist.

13. Injektionskartusche zum Beladen mit einer weichen Intraokularlinse mittels der Vorrichtung zum Laden gemäß einem der Ansprüche 1 bis 11, umfassend eine Aufnahmekammer für eine Linse in gefalteter Form, die durch eine Injektionskanüle zum Injizieren der gefalteten Linse verlängert ist, **dadurch gekennzeichnet, dass** die Aufnahmekammer (71) aus einem einzigen Rohr (72) gebildet ist und einen kegelstumpfförmigen Eingangsbereich (711) der Aufnahmekammer (7a) aufweist, der die konischen Endteile (331, 332) der Arme aufnehmen kann, die sich im Endteilstück (13) des röhrenförmigen Elements erstrecken.

14. Kartusche gemäß Anspruch 13, **dadurch gekennzeichnet, dass** die Aufnahmekammer (71) ein kegelstumpfförmiges Teilstück (711) umfasst, das auf der einen Seite durch die Einlassöffnung (71a) der Aufnahmekammer begrenzt wird und sich auf der anderen Seite durch ein zylindrisches Teilstück verlängert (712).

15. Kartusche gemäß Anspruch 13 oder 14, **dadurch gekennzeichnet, dass** dieses Rohr auf seiner äußeren Wandung einen Längsflügel (74) umfasst, der sich radial nach außen erstreckt.

16. Einheit, umfassend eine Ladevorrichtung gemäß einem der Ansprüche 1 bis 12 und eine Injektionskartusche (7, 107), umfassend eine Aufnahmekammer (71, 171) und eine Injektionskanüle (73, 173).

17. Einheit gemäß Anspruch 16, **dadurch gekennzeichnet, dass** sie eine Injektionskartusche (7) gemäß einem der Ansprüche 13 bis 15 umfasst.

## Claims

1. Device for loading a soft intraocular lens into an injection cartridge comprising a chamber for receiving a lens and an injection cannula, comprising:
- a tubular element (1, 101) having an internal passage (10, 110), said passage comprising a receiving section (11, 111) for a lens (9) in the unfolded state, an intermediate truncated folding section (12, 112) and an end section (13, 113) which is able to receive an injection cartridge in a removable manner (7, 107) in such a way that the intermediate truncated folding section leads to the receiving chamber (71, 171) of the injection cartridge,
- a support element (3) mounted in said receiving section which is able to support a lens in an unfolded state, and
- a pusher (5) comprising a rod (51) mounted in a mobile manner in said internal passage, capable of pushing a lens (9) arranged in the receiving section through the intermediate truncated folding section to fold said lens progressively, then into the receiving chamber of an injection cartridge placed in the end section.

2. Device according to claim 1, **characterised in that** it comprises braking means (54, 40) creating at least return resistance at the rear of the rod (51) of the pusher in said internal passage (10, 110), wherein said end section (13, 113) is able to receive, in a removable manner, retaining means (6, 106) extending in the internal passage up to its receiving section in order to longitudinally block a lens (9) arranged in the latter against the free end (51a) of the rod of the pusher.

3. Device according to claim 2, **characterised in that** said braking means create a return blockage at the rear of the rod (51) of the pusher (5) in the internal passage (10, 110) and resistance to the progression of the rod (51) of the pusher in the internal passage.

4. Device according to one of claims 1 to 3, **characterised in that** the internal passage (10, 110) of the tubular element (1, 101) comprises an open distal end (10b, 110b) through which an injection cartridge (7, 107) is slotted into the cylindrical end section (13, 113), wherein the tubular element is equipped with a longitudinal slot (15, 115) leading to said end section and extending up to said distal end to allow the passage of the blade (74, 174) of an injection cartridge.

5. Device according to claim 4, **characterised in that** the longitudinal slot (15, 115) is of bayonet type in such a way as to allow blockage of an injection cartridge (7, 107) in the end section (13, 113) through engagement of its blade (74, 174) in said longitudinal slot.

6. Device according to one of claims 1 to 5, **characterised in that** the internal passage (10, 110) of the tubular element comprises an open proximal end (10a, 110a) through which the support element (3) is slotted into the receiving section (11, 111), and the support element (3) is formed of a point (32, 33) having a head (31) which is slotted into the cylindrical receiving section (11, 111), wherein the point is equipped with a longitudinal slot (34) defining an upper branch (35) and a lower branch (36), between which a lens (9) can be received, said head of the point having a channel (39) leading between said branches and in which the rod (51) of the pusher is mounted in a mobile manner.

7. Device according to claims 2 and 6, **characterised in that** the retaining means comprise a nozzle (6, 106) comprising a rod (62) which is able to be slotted into the internal passage (10, 110) by its open distal end (10b, 110b) and is inserted between the two branches (35, 36) of the support element in order to longitudinally block a lens in the receiving section (11, 111) between the free end (51a) of its rod and the free end (62a) of the rod of the pusher.

8. Device according to claim 6 or 7, **characterised in that** said point (32, 33) has a conical end extending into the truncated folding section (12, 112) of the internal passage, the external faces of the corresponding end conical parts (331, 332) of the branches (35, 36) fitting the truncated wall of the folding section closely, the opposite internal faces (36a, 331a) of said end conical parts being shaped in such a way as to form guiding surfaces for the folding of the lens.

9. Device according to claim 8, **characterised in that** the slot (34) of the point is shifted with respect to the longitudinal axis of the point, the end conical part (332) of the lower branch (36) has a concave internal face (36a), the end conical part (331) of the upper branch (35) having a convex internal face (331a).

10. Device according to claim 8 or 9, **characterised in that** the end conical parts (331, 332) of the branches extend into the end section (13) of the tubular element and are able to be inserted into the entry truncated section (711) of the receiving chamber (71) of an injection cartridge (7) placed in said end section (13).

11. Device according to one of claims 6 to 10, **characterised in that** the tubular element (1, 101) comprises lateral openings (17) leading into the receiving section (11, 111), between the branches (35, 36) of the tubular element, to enable the passage of a sterilising agent, lubricants and/or a tool for the positioning of the haptic part of the lens with respect to the rod of the retaining nozzle.

12. Device according to one of claims 1 to 11, **characterised in that** it is loaded with a soft intraocular lens (9) and packaged in a sterile case.

13. Injection cartridge designed to be loaded with a soft intraocular lens by means of the loading device according to one of claims 1 to 11, comprising a receiving chamber for a lens in the folded state, extended by an injection cannula for the injection of the folded lens, **characterised in that** the receiving chamber (71) is formed of a single tube (72) and has a truncated entry section (711) of the receiving chamber (71) which is able to receive the conical end parts (331, 332) of the branches extending into the end section (13) of the tubular element.

14. Cartridge according to claim 13, **characterised in that** the receiving chamber (71) comprises a truncated section (711) which is delimited on one side by the entry opening (71a) of the receiving chamber and extending on the other side through a cylindrical section (712).

15. Cartridge according to claim 13 or 14, **characterised in that** said tube comprises a longitudinal blade (74) on its external wall which extends radially towards the exterior.

16. Assembly comprising a loading device according to one of claims 1 to 12 and an injection cartridge (7, 107) having a receiving chamber (71, 171) and an injection cannula (73, 173).

17. Assembly according to claim 16, **characterised in that** it has an injection cartridge (7) according to one of claims 13 to 15.
